# EUROPEAN PATENT APPLICATION

(11) **EP 4 015 053 A1**
(43) Date of publication of application: **22.06.2022**
(21) Application number: 20215324.3
(22) Date of filing: 18.12.2020
(51) Int. Cl.: A63F 13/212, A63F 13/245, A63F 13/428, A63F 13/5255, A63F 13/816, A63B 24/00, G16H 20/30

(54) **A SYSTEM TO INTERFACE A PHYSICAL TRAINING DEVICE AND COMMERCIAL VIDEOGAMES**

(71) Applicant: ETH Zurich, 8092 Zurich (CH)
(72) Inventor: HAUFE, Florian, 8037 Zürich (CH); XILOYANNIS, Michele, 8057 Zürich (CH)

(57) **Abstract**

The present invention relates to an interface arrangement (1) allowing to interface a physical training device (2), such as a wearable robot, with a videogame installation (3), and comprising a movement mapping unit (13) allowing to associate movements of the player to original commands of a given videogame. The present invention also discloses a method to allow or induce people to practice specific physical exercises while controlling a videogame.

## Description

### Technical domain

The present invention relates to a system allowing or inducing a human being to perform some specific movements, while playing to a commercial videogame. The specific movements can be part of a predetermined physical program, such as a physical training or a rehabilitation program. In particular, the present invention provides an interface arrangement allowing to interconnect a physical training device, comprising a set of movement sensors, to a selection of commercial videogames so that a human being can execute some predetermined movements.

### Related art

In the field of physiotherapy, some rehabilitation programs exist to help patients progressively recovering their physical capabilities. However, these programs, while requesting the patient making efforts, are usually repetitive and may be perceived as boring and non-encouraging. Thus, the success of a rehabilitation program largely depends on the patient determination and perseverance. A significant number of patients give up before the end of their rehabilitation program, leading to uncomplete recovery. Complications or recurrences of the failure.

Some videogames have been specifically developed for this purpose, so that the patient is encouraged performing all the necessary exercises. The diversity of such videogames, as well as their quality, remain however limited. The patient may thus remain undermotivated to perform the rehabilitation program. In addition, due to the limited diversity of medical videogames, it is still possible that no existing medical videogame are adapted for a given rehabilitation program.

Some healthy people may refrain practicing some physical exercises, by lack of motivation or lack of available proper equipment. Some muscles weaknesses may thus appear and eventually result in physical impairments, in particular regarding the articulations or the back, from the lumbar to cervical. Preventive physical exercise may thus be beneficial, at least for some people at risk.

It is also the case that young healthy people spend a large part of their free time playing to videogames, which results in a lack of physical activity and muscles weaknesses. While preventing them to play may results in tensions or conflicts, it may be beneficial allowing them keep playing and at the same time executing some physical activity.

There is thus a room to better exploits the existing videogames for motivating people to execute some physical movements, either in the framework of medical program or merely for leisure activities.

### Short disclosure of the invention

An aim of the present invention is the provision of an interface arrangement that overcomes the shortcomings and limitations above-mentioned. It is in particular the aim of the present invention to provide an interface arrangement adapted to allow interaction between an existing commercial videogame and a physical training device such as a wearable robot or a set of wearable sensors, so that a user is allowed or motivated to make exercise. It is furthermore the aim of the present invention allowing or inducing a user to perform specific predetermined movements while playing to a commercial videogame .

It is also an aim to better motivate patients in need of rehabilitation program, or preventing exercises by allowing them access to a large variety of existing videogames.

It is also an object of the present invention to prevent or limit the physical impairment by lack of physical activity.

Another aim is to provide a valuable and flexible tool for the physiotherapists, offering a better variety of rehabilitation programs and improve the patient satisfaction.

Another aim of the invention is the provision of a method of rehabilitation or physical performances improvement based on existing videogames.

According to the invention, these aims are attained by the object of the attached independent claims, and further detailed in the dependant claims.

### Short description of the drawings

Exemplar embodiments of the invention are disclosed in the description and illustrated by the following drawings :
- Figure 1 : Schematic representation of the interface arrangement of the present invention;
- Figure 2: scheme showing the method according to an example of the present invention.

### Examples of embodiments of the present invention

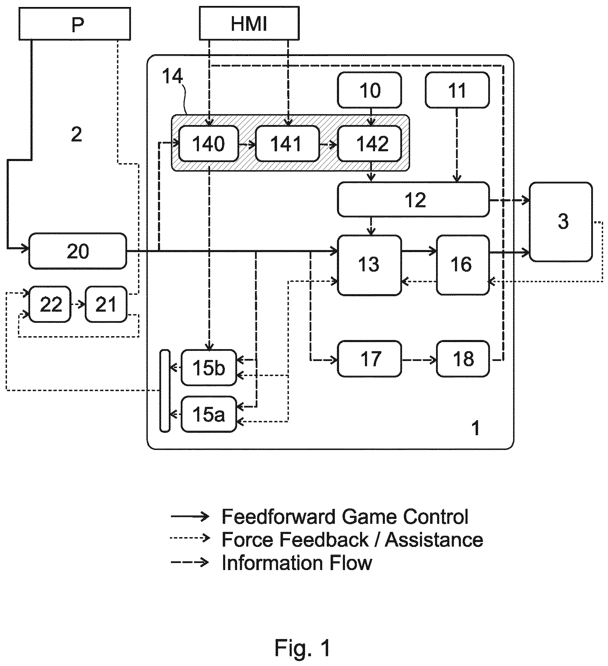

With reference to figure 1, the interface arrangement 1 is connectable to a physical training device 2 and to a videogame installation 3 so as to allow a player P to interact with a variety of different commercial videogames by the means of the physical training device 2. The interface 1 is adaptable to a variety of different physical training device 2.

A physical training device here-mentioned denotes any device comprising at least one movement sensor 20 allowing to identify at least one predetermined movement of the player. A physical training device according to the present invention can be for example a set of movement sensors arranged on the body of the player to recognize some predetermined movements, also known as body-worn sensors. Although the movement sensors can be placed directly on the body, they can alternatively be combined to wearable elements such as gloves, belts, or any other suitable wearable element that the player can place at any part of his body, including the arms, the legs, the head. Alternatively or in addition, a physical training device according to the present invention can comprise one or more remote movement sensors arranged at distance from the player, such as cameras or other optical sensors. A physical training device 1 includes the wearable robots.

A wearable robot is understood as a device placed on the body and allowing to determine movements of the body. In particular a wearable robot allows to determine one or more of the directions, the amplitude, the strength, the duration and the speed of a body movement. movement sensors 20 such as accelerometers, stretch sensors or other movement sensors may be used to this end. Other sensors such as external optical sensors may also be used in addition to the body-worn sensors. In addition, a wearable robot according to the present disclosure may comprises means for providing to the user haptic effects such as force resistance, vibrations, and any other physical sensations. A wearable robot according to the present disclosure thus comprises at least one actuator 21, and at least one actuator controller 22 to provide and control such physical or haptic effects. The wearable robot 2 may be associated to a part of the body such as a wrist, an arm, a leg, or to several parts of the body. An actuator can be an electrical motor, a pneumatic actuator, a vibrating device or any other suitable actuator.

Throughout the present disclosure, a "player" denotes a human being actively interacting with a videogame so as to control the videogame. The player may be a patient having physical impairment and in need of rehabilitation or prophylaxis physical exercises to prevent failure. A player can also denote a healthy people aiming at specific physical training.

Throughout the present disclosure, a "user" denotes a human being interacting with the setting of the interface arrangement so that proper movements are executed by the player when playing to a selected videogame. Although the user may be the player himself, he can be a different person, such as a physiotherapist, a coach or any other relevant person.

Where the male gender "he", "his" and related, is used, it also includes the female counterpart "she", "her" and related.

A videogame installation 3 is understood as comprising at least one executable interactive videogame, means for reading and executing the videogame, a display to visualise a videogame and at least one set of original commands allowing the player P to interact with the videogame. A display denotes here any means to visualise a videogame such as a screen a pair of virtual-reality glasses or a device allowing augmented reality. The executable videogame may be stored on a readable storage means such as a DVD, Blueray or equivalent. Alternatively, the executable videogame may be stored on a remote server. Means for reading and executing the videogame may be a computer or any other dedicated electronic device such as a gaming box, a gaming console. The set of original commands denotes any human machine interface device (HMI) such as a joystick or a keyboard, commonly used by a player P. The original commands are preferably manual devices which are exclusively activated by the fingers of a player P and do not necessitate specific body-gestures. Original commands being manipulated by the player P have a predetermined executable action in the videogame. In a more general way, an original command is exclusively dedicated to the command of the commercial videogame and is not conceived to be interfaced with a physical training device. A videogame installation (3) may include or denotes a simulator, provided that the diversity of movement is note limited by the design of the simulator and that a variety of videogames can be selected.

The interface arrangement 1 of the present disclosure allows a user to mapp some movement sensed by the movement sensors 20 of the physical training device 2 to a variety of different original commands of videogames. The interface arrangement 1 may allow the player piloting all the commands or only a part of the commands of the videogame using specific dedicated movements so that it is no longer necessary to manipulate the set of original commands of the videogame. Under these conditions, all the commands of a given videogame, or only a part of them, may be executed through the interface arrangement 1. It is understood that while the original commands are still involved in the control of the videogame, they are not directly activated by the player but through the interface arrangement 1. It is also understood that any movement may be chosen to play the videogame independently on the corresponding original command.

According to an embodiment, the interface arrangement 1 may be plugged or connected to the commercial videogame so as to replace one or several original command of the videogame. The interface arrangement 1 may for example integrate the original command or being integrated to the original command.

Alternatively, the interface arrangement 1 may come in addition to the set of original commands of the videogame. This allows for example thatonly a part of the commands of the videogame are executed through the interface arrangement 1. This does not exclude that both set of original commands and the interface arrangement 1 are used in parallel, allowing to interact with the videogame either through the interface arrangement 1 or with the set of original commands. Such a disposition is for example advantageous in case of multiplayers videogame, only one of those wears a wearable robot 2.

The mapping of a given gesture to a command of the videogame is performed by means of a physiological converter unit 12. The physiological converter unit 12 allows basically to replace one or several original commands of the videogame by specific gestures of the player P. It furthermore allows to identify which original command of a given videogame is best replaced by a gesture of the player P. For example, certain original commands of a given videogame having specific frequencies may be selected to be performed through the interface arrangement 1 so that some gestures are executed at the expected frequency. When it is determined that a frequent specific gesture needs to be performed during the videogame, the most frequently used original command of the videogame may be selected to be executed through the interface arrangement 1 instead of the original set of commands. The same applies for the duration of the commands. In case a given gesture should be maintained for a while, a long original command is better selected to be replaced by a gesture of the player P. The selection of the original command to be performed through the interface arrangement 1 thus depends on the videogame itself, the parameters of the commands such as its frequency, amplitude, strength, speed, and duration, and on the characteristics of the movements to be performed.

The physiological converter unit 12 may alternatively or in addition determine that a given original command is fully replaced by a gesture of the player P, meaning that all the characteristics of the original command, including its speed, duration, frequency, amplitude and strength, are integrally performed by a gesture of the player. Alternatively, the physiological converter unit 12 may allow an adapted conversion of an original command to a gesture. For example, a reduced speed of a gesture may allow to perform a command which is originally faster. Alternatively or in addition, a reduced duration of a gesture may allow an originally longer command. The same applies for the strength, the amplitude, the frequency and any other parameters of a given gesture. Such an optimisation of the conversion allows to adapt the gestures to the physical capabilities and/or the physical performance objectives of the player P. In other words, the efforts made by the players may be minimized compared to the efforts requested by the original command. It is also possible to increase the physical efforts of the players for one or several specific gestures, compared to the corresponding efforts of the original command.

The physiological converter unit 12 may in addition determine the degree of the haptic effect corresponding to a given gesture of the player P. For example, in case a force resistance is provided to the player P, the intensity or duration or gradation of the force resistance may be optimized, meaning that one or more of its parameters can be minimized so that haptic feedback is not perceived as painful by the player P, or increased to improve his physical performances.

The physiological converter unit 12 may alternatively or in addition select a videogame or a short list of videogames based on their original command characteristics and on the expected movement to be performed by a player P. For example, where ample movements need to be performed by a player P, the physiological converter unit 12 provides a list of videogames having or allowing at least one ample original command. This ample original command will then be converted or associated to a command movement to be performed by the player P. In other words, the physiological converter unit 12 discards from a predetermined list all the videogames which do not propose sufficiently ample commands. More generally, the physiological converter unit 12 discards all the videogames the commands of which do not satisfy the movement characteristics to be performed by the player P. In case no existing videogames comprises the command having the expected characteristics, the physiological converter unit 12 may select a non-optimized videogame and optimize the conversion of at least one of its original command so that an adequate movement is performed by the player P. For example, in case a given duration of movement is required and no videogame of the predetermined list comprises an original command having the appropriate duration, the physiological converter unit 12 may request from the player P a movement longer than the duration of the original command. In other words, the physiological converter unit 12 provides a videogame or a list of videogames, the original command of which can be converted or associated to an expected movement of the player P, either with or without optimization.

According to a preferred embodiment, the optimization is performed by the physiological converter unit 12 so as to minimize a cost function
f(x)
which is formulated to measure the distance between a set of predetermined movements to be executed by the player P and the set of original commands of the videogame. The cost function f(x) includes several characteristics such as the frequency of occurrence of a command, its duration, its amplitude, its speed, and any other relevant parameter. The optimization also considers some accessibility constraints determined by an accessibility function
g(x),
which is formulated to quantify the accessibility of the videogame by a player P. The accessibility function g(x) may thus consider parameters related to the physical capabilities of the player P or his degree of training, or any other relevant parameter. The accessibility function g(x) is subject to un upper limit "c" so that
g(x) < c.

In the cost function f(x) and the accessibility function g(x), the variable "x" denotes the mapping between the movements of the player P and the corresponding commands of the videogame. An optimized mapping "xₒₚₜ" can thus be determined according to the above-mentioned functions f(x) and g(x) for each videogame of a predetermined list of videogames. The videogames may thus be ranked according to their optimized gesture mapping. Weight or ponderation may in addition be considered, for example to reflect the players preferences, his age or other parameters.

The predetermined list of videogames is preferably stored in a videogame database 11. The videogame database 11 may comprise several executable videogames. It is however not necessary that the videogame database 11 comprises as such the commercially available videogames. It is enough that the videogame database 11 comprises data allowing to identify a commercially available videogame as well as its original command characteristics. The videogames may be referenced according to their style or type, or degree of physical move it requires, or any other parameters. The videogame database 11 is readable and searchable in a way to identify the videogame the most suitable to make the player executing some predetermined gestures. Although the selection or a preselection of videogames may be performed manually by a user or the player himself, it is preferably done automatically by the physiological converter unit 12 or any other unit, based at least on the characteristics of the gestures to be performed. The selection of a videogame may also be based on other parameters such as the player profile and preferences. The selection of a given videogame or a list of appropriate videogames is thus preferably operated via an algorithm.

The interface arrangement 1 further comprises a movement mapping unit 13 allowing to convert at least some of the gestures of the player to commands of the videogame. In particular, the movement mapping unit 13 recognizes the gestures of the players corresponding to the predetermined gestures which replace the original commands of the videogame. The movement sensors 20 allow to determine the movements of the player and identify some predetermined movements that should be executed for a given command. When a movement is recognized as corresponding to a predetermined movement by the movement mapping unit 13, the corresponding command is executed. The corresponding command denotes here the videogame command already identified by the physiological converter unit 12 to be replaced by a movement of the player. The movement mapping unit 13 thus allows to recognize a movement of the player and to determine at least one of its speed, strength, amplitude, duration or direction. A correct movement is identified when some of its parameters or all of its parameters correspond to the movement characteristics defined by the physiological converter unit 12 to execute a command of the videogame. The correctness of the movement may be determined by a percentage of execution compared to the requested movement. For example, when a movement of a specific amplitude is requested to execute a command, it may be considered that the movement is correct if its amplitude is higher than 80% or 90% or 95% of the requested amplitude. The same applies to the strength, the speed, the duration, the direction and any other parameters. A combination of several parameters may be used to determine the correctness of the movement. For example, a combination of the speed and the direction of a movement may be considered. The requested movement may be already an optimized movement or not.

When the movement of the player is not recognized as the correct movement, then the expected command is not executed.

The movement mapping unit 13 allows in addition to provide the haptic effects to the players through the physical training device 2 if equipped with the corresponding actuators The type of effect, as well as its intensity, are those determined by the physiological converter unit 12. A haptic effect may be provided in case of correct movement, or in case of incorrect movement, or in both cases. Different haptic effects may be provided in case of correct or incorrect movement.

As a concrete example, the videogame may be a car race. The original command dedicated to the driving speed can be replaced by a movement of an arm of the player by the physiological converter unit 12. By rising the arm in front of him and maintaining it at a horizontal position, the player may be able to pilot a car at a given speed. When lowering his arm, the car slows down. When raising his arm above his head the care accelerates at its maximal speed. Thus, piloting the car obliges the player maintaining his arm at a given position. The arm gesture of the player is converted to a speed command to the videogame thanks to the movement mapping unit 13. In case of collision, some haptic effects such as vibrations may be applied to the arm of the player. The car race may have been selected by the physiological converter unit 12 among a list of several videogames, to be the most appropriate for maintaining the arm at a horizontal position for a while.

The haptic effects can be transmitted to the physical training device 2 by means of a haptic effect module 15a connected to the actuators of the physical training device 2, where applicable. An assistance module 15b may in addition be considered so as to reduce or minimize the physical effort of the player for a given movement. It can alternatively be used to resist the player's effort and make his task harder. Such an assistance is provided according to the optimized movement determined via the physiological converter unit 12. The assistance module 15b is also connected to the actuators of the wearable robot 2.

The interface arrangement 1 may further comprise a physiological exercises database 10, comprising a plurality of movements or combination of movements. Movements may be referenced in the physiological exercises database 10 at least by their type, such as movement of wrist, arms, legs, hands and so on. Additional parameters may be predetermined or programmable. For example, the degree of a given movement type may be selected among a list of predetermined degrees such as low intensity, fair intensity, high intensity and so on. It can alternatively be tunable according to the needs. For example, a fast vertical move of an arm may be selected, either within a predetermined intensity scale allowing a fast vertical move, or by tuning on demand the speed of a predetermined vertical move. The physiological exercises database 10 thus allows to select specific movement to be executed by the player and to implement them in the physiological converter unit 12 so that it can be used as a videogame command. The physiological exercises database 10 may in addition comprise predetermined series of movements, combined movements, and any other movement arrangement. Some movement may be manually selected by a user out of the physiological exercises database **10** so that the physiological converter unit **12** determines the proper videogames and the proper original command to be replaced by, or associated to, the selected movement.

According to an embodiment, the movement or a combination of movements is automatically selected out of the physiological exercises database **10** based on a predetermined training program generated by a training unit **14.** The training unit **14** may comprise a player assessment unit **140** allowing to determine one or more of the physical capabilities of the player, the physical performance objectives and the preferences of the player. The physical capabilities may refer to average data related to age, gender, profession, and any other demographic data. Based on the corresponding information related to the player, a profile corresponding to an average profile is built. Alternatively, the physical capabilities can be individually determined for a given player considering for example his daily physical activity or some results of physical tests. The player assessment unit **140** may in addition consider some clinical or physical weaknesses. This is advantageously applied for patients needing a rehabilitation program. It is however not mandatory since any player may use the interface arrangement 1, including healthy players looking for improved physical performances. The player assessment unit **140** may comprise or be connected to a graphical user interface allowing to directly upload data. Alternatively or in addition, the player assessment unit **140** can comprise or be connected to body-worn sensors so that the physical behavior of the player can be determined. According to a preferred embodiment, the body-worn sensors are those of the wearable robot **2** to which is connected the interface arrangement 1. According to a specific embodiment, a test videogame may be offered to a player to determine the characteristics of his movements through the physical training device **2.**

The player assessment unit **140** may also comprise a training goal unit **141** allowing to define a physical performance objective such as improving the strength of the hand up to a predetermined force value or improving the amplitude of move of a shoulder up to a predetermined amplitude value. The training goal unit **141** may be manually loaded with specific performance objectives by the player himself or by another user. Such another user may be a coach, a therapist, or any other person. A graphic interface may be used to load the physical performance objectives. Predetermined menus or performance scales may be available and selected by the user.

The player assessment unit **140** may in addition comprise a training plan unit **142** automatically determining the proper movement program to be implemented for reaching the physical performance objectives selected through the training goal unit **141.** Such a movement program can select the predetermined movements or combination of movements stored in the physiological exercises database **10.** An algorithm can help determining the proper movements or combination of movements to be selected, based on several parameters, including the parameters available in the player assessment unit **140** such as the age, the general physical capabilities of the player.

The interface arrangement **1** may in addition be provided with a connection module **16** allowing to interact with the videogame installation **3.** The connection modules **16** handles a bidirectional protocol. It is preferable an I/O connection module. Although a proprietary software may be used, the connection module **16** can include some open source software known to one skilled in the art.

The interface arrangement **1** may in addition comprise a datalogger **17** allowing to store some data related to the played videogames, combined with the corresponding scores, to the player himself, including his training programs, past or ongoing, to the movement sensors **20,** to the output and calculated performances related to the played games, and any other data.

The interface arrangement 1 may in addition comprise a report generator **18,** connected to the datalogger **17** and/or any other unit of the disclosed interface arrangement 1. The report generator **18** provides reports on the player's performances, statistics related to physical progress or any other parameters resulting from the player's activity. This report is preferably human readable so that the player or another user can gain knowledge of it and follow his physical capabilities. The data mentioned in the report or part of these data may be updated to the player's profile, for example via the player assessment unit **140** or a manual interface. In addition or alternatively, the training goal may be adapted according to the results of the report, either automatically through the training goal unit **141** or manually by a user. In addition, an automatic training plan may be generated and suggested to the player based on the data of the report. An updated selection of videogames can be suggested or a new optimization of movements can be proposed, for example through the physiological converter unit **12.**

Although the interface arrangement **1** is described as an independent unit connectable to both a videogame installation **3** and a physical training device **2,** this does not exclude that the present interface arrangement is integrated to an existing device, either fully or partly. One or more of the elements above-described, including the videogame database **10,** the physiological converted unit **12,** the mapping unit **13,** the training unit **14,** the haptic feedback module **15a,** the assistance module **15b,** the datalogger **17** and the report generator **18,** may be integrated or combined to the videogame installation **3** or to the physical training device **2.** Also, some preexisting elements in the videogame installation **3** such as for example videogame libraries or dataloggers, may be adapted to operate according to the corresponding elements above-described.

It is in any case the aim of the present interface arrangement **1** to be adapted to a large variety of existing videogames and videogame installations so that specific movements of the player can be arranged for at least some of the videogame commands.

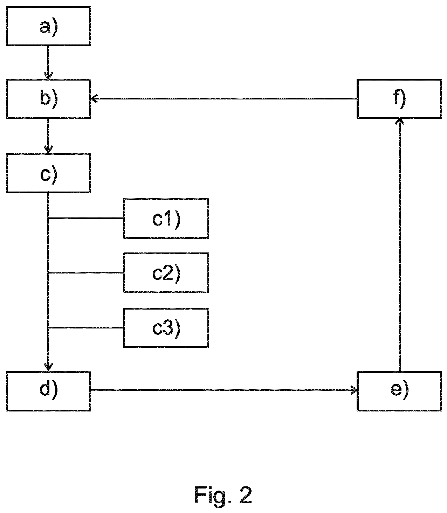

The present disclosure also relates to a method for converting at least one of the original commands of a variety of videogames to specific movements of a player. According to the present method, an existing videogame originally not conceived for particular physical moves can be played in a way that specific movements are required from the player. This is particularly convenient for motivating patients in need of rehabilitation programs and thus improve and accelerate the rehabilitation results. The method thus comprises an interfacing step a) (Fig. 2) of connecting an interface arrangement between a videogame installation **3** and a physical training device **2.**

The method further comprises a training determination step b) of determining a movement or a combination of movements the player needs to execute. The movement or combination of movements may be selected out of a physiological exercises database (10). The movement or combination thereof may be selected based on parameters including some player assessment data, some specific performance objectives to reach, some medical or physiological limitations, and any other relevant parameters. This data and parameters may be uploaded in specific units such as a player assessment unit **140,** a training goal unit **141** and a training plan unit **142.**

The method further comprises a physiological conversion step c) of interfacing the selected movement or combination of movements to a videogame. Step c) comprises a sub-step c1) of selecting at least one videogame from a videogame library. Such a videogame library may be stored in a videogame database **10** allowing to identify known videogames and their original commands. Step c) comprises a sub-step c2) of interfacing the movement or the combination of movements determined in step b) to at least one command of a videogame. The interfaced videogame is preferably the videogame selected in the sub-step c1). The step c) may in addition comprise a sub-step c3) of optimizing the movement or the combination of movements to be executed by a player either to minimize, reduce or increase the efforts of the player. The optimization may be performed based on a cost function f(x) and an accessibility function g(x) as described above. The physiological conversion step c), including one or more of the sub-steps c1), c2) and c3) may be performed by a physiological converter unit **12** allowing to convert an original videogame command into a movement.

The method further comprises a practicing step d) wherein the player executes the movement or combination of movements determined in step b) to play the videogame interfaced in step c). The movements may be recognize and analysed by a movement mapping unit **13** so that only a correct movement allows to execute a videogame command. The practicing step d) may further comprise a sub-step d1) of providing to the player haptic feedback.

The method also includes a reporting step e) of computing data and statistics related to the player activities and generating a human readable report showing the physical performances and or the physical capabilities of the player.

The method may in addition comprise an upgrade step f) of determining a new movement or combination of movements based on the report generated in step e) or based on any other data. Iteration of steps b), c), d) and e) can then be executed.

The present disclosure further relates to a kit or an equipment allowing for executing some specific movement or combination of movements. Such a kit may be self-usable by a player or a patient at home to practice specific physical move either for increasing muscles performance or for completing a rehabilitation program. The kit may alternatively be used at a medical center or rehabilitation center such as a physiotherapist consulting room, since it can allow very different patients executing different movements based on their own physical capabilities and preferences. It may alternatively be used in a training center, such as a sport training center, or in a playing center offering to players a large selection of videogames. The kit comprises a videogame installation **3,** at least one physical training device **2** and at least one interface arrangement **1** as described above. When comprising several different physical training device **2,** the kit may easily be adapted to different players for different movements. The interface arrangement **1** allows to associate original commands of a large variety of videogames to a large variety of physical training devices **2,** including wearable robots.

### reference symbols in the figures

- 1: Interface arrangement
- 10: Physiological exercice database
- 11: Videogame database
- 12: Physiological converter unit
- 13: Movement mapping unit
- 14: Training unit
- 140: Player assessment
- 141: Training goal unit
- 142: Training plan unit
- 15a: Haptic effect module
- 15b: Assistance module
- 16: Connection unit
- 17: Datalogger
- 18: Report generator
- 2: physical training device
- 20: Movement sensor
- 3: Videogame installation
- P: Player
- HMI: Human-machine interface

## Claims

1. An interface arrangement (1) comprising at least one connection point to a physical training device (2) and at least one connection point to a videogame installation (3), **characterized in that** said interface arrangement (1) comprises:
- a physiological exercises database (10), comprising a plurality of predetermined movements to be made by a player,
- a videogame database (11) comprising a list of videogames and the corresponding original commands,
- a physiological converter unit (12), allowing to select videogames out of the videogame database (11), to select one or more original commands of the selected videogame and to associate said one or more original commands to movements of the physiological exercises database (10) so that said one or more original commands of said videogame can be executed by said movement from the player,
- a movement mapping unit (13) allowing to convert said movement of a player into commands for said videogame.

2. Interface arrangement according to claim 1, further comprising a training unit (14) allowing to select one or more movements from the physiological exercises database (10).

3. Interface arrangement according to claim 2, wherein said training unit (14) comprises one or more of a player assessment unit (140), a training goal unit (141) and a training plan unit (142).

4. Interface arrangement according to one of claims 1 to 3, wherein said physiological converter unit (12) allows to optimize at least one of the parameters of the movements to be made by the player so that a videogame command is executed.

5. Interface arrangement according to claim 4, wherein the optimised parameters of the movements increase, decrease or minimize the efforts of the player when performing said movements to execute a videogame command.

6. Interface arrangement of one of claims 1 to 5, wherein the physical training device (2) is a wearable robot comprising at least one actuator, wherein the interface arrangement further comprising one or more of a haptic effect module (16a) allowing to provide a proper haptic feedback to the player, and an assistance module (15b) allowing to conjugate the player's efforts with some power from the wearable robot.

7. Interface arrangement according to one of claims 1 to 6, further comprising one or more of a datalogger (17) allowing to store data and statistics related to a player's activity, and a report generator (18) adapted for generating a human readable report related to a player's activity.

8. Interface arrangement according to one of claims 1 to 7, wherein said physical training device (2) comprises at least one body-worn sensor, at least one actuator (21) and at least one actuator controller (21).

9. Method for associating at least one original command of a variety of videogames to specific movement of a player, comprising :
- An interfacing step a) of connecting an interface arrangement between a physical training device (2) and a videogame installation (3),
- A training determination step b) of determining a movement or a combination of movements to be executed by the player,
- A physiological conversion step c) allowing to associate an original command of an existing videogame to the movement or combination of movement determined in step b), and
- A training step d) of performing the movement so as to execute the command of said videogame associated to an original command in step c).

10. Method according to claim 9, further comprising a reporting step e) of generating a human readable report based on the playing activities of the player, so as to determine at least some of his physical capabilities.

11. Method according to claims 9 or 10, further comprising an upgrade step f) of determining new movement or combination of movements based on the physical capabilities of a player and reiterate one or more of steps b), c), d), and e).

12. Method according to one of claim 9 to 11, wherein the step c) comprises one or more of the sub-steps c1) of selecting at least one videogame from a videogame library, a sub-step c2) of interfacing the movement or the combination of movements determined in step b) to at least one command of a videogame and a sub-step c3) of optimizing the movement or the combination of movements to be executed by a player.

13. Method according to claim 12, wherein the optimization of sub-step c3) includes minimizing a cost function f(x), formulated to measure the distance between a predetermined set of movements to be executed by the player P and a set of original commands of the videogame and determine an accessibility function g(x) formulated to quantify the accessibility of the videogame by a player **P** so that g(x) remains under an upper a predetermined limit "c", wherein (x) denotes the mapping between the gesture of the player **P** and the corresponding command of the videogame.

14. A kit or an equipment adapted for practicing predetermined movements, comprising :
- a physical training device (2) having at least one movement sensor (20), at least one actuator (21) and at least one actuator controller (22),
- a videogame installation (3), and
- at least one interface arrangement (1) according to one of claims 1 to 8.
